# FASCICULE DE BREVET EUROPEEN

(11) **EP 4 267 290 B1**
(45) Date de publication et mention de la délivrance du brevet: **12.02.2025**
(21) Numéro de dépôt: 21824408.5
(22) Date de dépôt: 14.12.2021
(51) Int. Cl.: B01J 4/00, B01J 10/00, B01J 19/26

(54) **PROCEDE D'OLIGOMERISATION DANS UN REACTEUR COMPRENANT UN DOUBLE DISTRIBUTEUR GAZ/LIQUIDE**
VERFAHREN ZUR OLIGOMERISIERUNG IN EINEM REAKTOR MIT EINEM DOPPELGAS-FLÜSSIGKEITSVERTEILER
METHOD FOR OLIGOMERIZATION IN A REACTOR COMPRISING A DUAL GAS-LIQUID DISTRIBUTOR

(30) Priorité: 23.12.2020 FR 2014021
(43) Date de publication de la demande: 01.11.2023
(73) Titulaire: IFP Energies nouvelles, 92500 Rueil-Malmaison (FR)
(72) Inventeur: RAYNAL, Ludovic, 92852 RUEIL-MALMAISON CEDEX (FR); VONNER, Alexandre, 92852 RUEIL-MALMAISON CEDEX (FR); MAXIMIANO RAIMUNDO, Pedro, 92852 RUEIL-MALMAISON CEDEX (FR)
(74) Mandataire: IFP Energies nouvelles
(86) Numéro de dépôt international: PCT/EP2021/085590
(87) Numéro de publication internationale: WO 2022/136013

(56) Documents cités:
- EP-A1- 2 318 129
- EP-B1- 2 318 129
- WO-A1-2019/011806
- CN-A- 110 898 698
- US-A1- 2011 137 083
- US-A1- 2018 009 662

## Description

### DOMAINE TECHNIQUE

La présente invention concerne le domaine des réacteurs gaz/liquide permettant l'oligomérisation d'oléfines en oléfines linéaires par catalyse homogène.

L'invention concerne également l'utilisation du réacteur gaz/liquide dans un procédé d'oligomérisation d'une charge oléfinique gazeuse, de préférence d'éthylène gazeux, en alpha-oléfines linéaires telles que le but-1-ène, le hex-1-ène, ou l'oct-1-ène ou un mélange d'alpha-oléfines linéaires.

### ART ANTERIEUR

L'invention concerne le domaine des réacteurs gaz/liquide encore appelés colonne à bulles, ainsi que leur mise en œuvre dans un procédé d'oligomérisation d'une charge oléfinique, de préférence de l'éthylène. Un inconvénient rencontré lors de la mise en œuvre de tels réacteurs dans des procédés d'oligomérisation de l'éthylène est la gestion du ciel gazeux, correspondant à la partie supérieure du réacteur à l'état gazeux. Ledit ciel gazeux comprend les composés gazeux peu solubles dans la phase liquide, des composés partiellement solubles dans le liquide mais inertes, ainsi que de l'éthylène gazeux non dissout dans ledit liquide. Le passage de l'éthylène gazeux de la partie inférieure liquide de l'enceinte réactionnelle vers le ciel gazeux est un phénomène appelé perçage. Or le ciel gazeux est purgé afin d'éliminer lesdits composés gazeux. Lorsque la quantité d'éthylène gazeux présente dans le ciel gazeux est importante, la purge du ciel gazeux entraine une perte en éthylène non négligeable ce qui nuit à la productivité et au coût du procédé d'oligomérisation. De plus, un phénomène de perçage important signifie que beaucoup d'éthylène gazeux n'a pas été dissout dans la phase liquide et donc n'a pas pu réagir ce qui nuit à la productivité et à la sélectivité du procédé d'oligomérisation.

Afin d'améliorer l'efficacité du procédé d'oligomérisation en termes notamment de productivité et de coût, il est donc indispensable de limiter le phénomène de perçage de l'éthylène afin d'améliorer sa conversion dans ledit procédé tout en conservant une bonne sélectivité en alpha oléfines linéaires souhaitées.

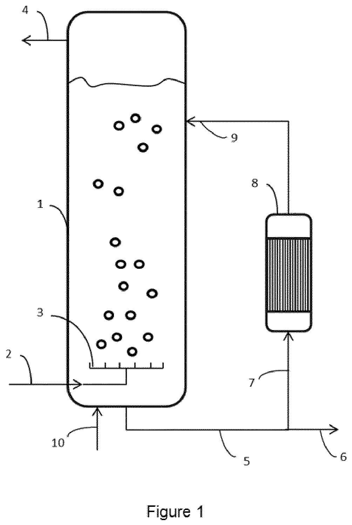

Les procédés de l'art antérieur mettant en œuvre un réacteur gaz/liquide, tel qu'illustré à la figure 1, ne permettent pas de limiter la perte en éthylène gazeux et la purge du ciel gazeux entraine une sortie d'éthylène gazeux du réacteur néfaste pour le rendement et le coût du procédé.

La demanderesse a décrit des procédés dans les demandes WO2019/011806 et WO2019/011609 permettant d'augmenter la surface de contact entre la partie supérieure de la fraction liquide et le ciel gazeux par l'intermédiaire de moyen de dispersion ou de vortex afin de favoriser le passage de l'éthylène contenu dans le ciel gazeux vers la phase liquide au niveau de l'interface liquide/gaz. Ces procédés ne permettent pas de limiter le phénomène de perçage et ne sont pas suffisants lorsque la quantité d'éthylène dans le ciel gazeux est importante du fait d'un fort taux de perçage.

De plus lors de ces recherches, la demanderesse a constaté que dans un réacteur fonctionnant à débit constant d'éthylène gazeux injecté, la quantité d'éthylène dissout et le phénomène de perçage sont dépendants de la taille des bulles d'éthylène gazeux injecté. Le temps dédié à la dissolution de l'éthylène gazeux dans la phase liquide correspond au temps de parcours des bulles dans la hauteur liquide, qui est imposée par les conditions opératoires et la hauteur du réacteur. La quantité d'éthylène gazeux dissoute par unité de temps est proportionnelle à la surface de contact entre les phases gaz et liquide. Plus les bulles sont grosses, plus leur ratio surface/volume est faible, plus le temps nécessaire à la dissolution est long, ce qui, augmente le phénomène de perçage pour une hauteur de la phase liquide donnée.

La demanderesse a découvert qu'il est possible d'améliorer la conversion d'oléfine(s), tout en conservant une sélectivité élevée en oléfine(s) linéaire(s) recherchée(s), et notamment en alpha-oléfine(s), en limitant les phénomènes de perçage au moyen d'un réacteur gaz/liquide particulier d'oligomérisation d'une charge oléfinique gazeuse, en particulier d'éthylène gazeux, comprenant un dispositif d'injection d'éthylène gazeux et un dispositif d'injection de liquide, lesdits dispositifs d'injection étant avantageusement agencés de manière à ce que l'injection du liquide puisse entrainer une diminution de la taille des bulles d'éthylène, par cisaillement, lors de l'injection de l'éthylène gazeux. Le réacteur gaz/liquide selon la présente invention peut être utilisé pour toute charge oléfinique gazeuse injectée dans une phase liquide.

### DESCRIPTION SOMMAIRE DE L'INVENTION

La présente invention concerne un procédé utilisant un réacteur gaz/liquide d'oligomérisation d'une charge oléfinique gazeuse comprenant un dispositif d'injection de gaz (3) et un dispositif d'injection de liquide (12), lesdits dispositifs d'injection étant agencés de manière à ce que l'injection du liquide puisse entrainer une diminution par cisaillement de la taille des bulles lors de l'injection de la charge oléfinique gazeuse.

De préférence, le dispositif d'injection de gaz (3) comprend au moins un orifice d'injection de gaz et le dispositif d'injection de liquide (12) comprend au moins un orifice d'injection de liquide, chaque orifice d'injection de gaz étant situé à proximité d'un orifice du dispositif d'injection de liquide (11) et positionné de manière à ce que la trajectoire d'injection du gaz soit dans le plan de la trajectoire d'injection du liquide.

De préférence, les orifices d'injection du liquide et les orifices d'injection de gaz sont de forme circulaire, et dans lequel les orifices d'injection du liquide présentent un diamètre supérieur ou égal au diamètre des orifices d'injection de gaz.

De préférence, au moins un orifice d'injection de gaz et au moins un orifice d'injection de liquide sont positionnés en regard l'un par rapport à l'autre selon un angle compris entre 0° et 180°.

De préférence, les dispositifs d'injection de gaz et d'injection de liquide sont choisis parmi une conduite, un réseau de conduites, un distributeur multitubulaire, une plaque perforée, un tube cylindrique, un tube concentrique.

De préférence, le dispositif d'injection de gaz est un tube cylindrique ayant une forme d'anneau circulaire présentant des orifices d'injection et le dispositif d'injection de liquide est un tube cylindrique ayant une forme d'anneau circulaire présentant des orifices d'injection.

De préférence, le dispositif d'injection de gaz sous forme d'anneau circulaire présente un diamètre inférieur à celui du dispositif d'injection de liquide sous forme d'anneau circulaire, et dans lequel ledit dispositif d'injection de gaz se positionne à l'intérieur du dispositif d'injection de liquide sur un plan différent.

De préférence, un enchainement de plusieurs dispositifs d'injection de liquide et de gaz sous forme d'anneau circulaire de diamètres décroissants sont alternés de la périphérie vers le centre représenté par l'axe central du dispositif ayant le diamètre le plus grand, lesdits dispositifs étant positionnés de sorte qu'un orifice d'injection de gaz d'un dispositif d'injection de gaz soit positionné à proximité d'un orifice du dispositif d'injection de liquide adjacent de manière à ce que la trajectoire d'injection de liquide soit dans le même plan de la trajectoire d'injection du gaz afin de provoquer le cisaillement dudit gaz.

De préférence, les orifices des dispositifs d'injection de gaz et de liquide sont prolongés chacun par un tube (13, 15).

De préférence, le ou les tubes (13) du dispositif d'injection de gaz (3) présente un diamètre inférieur à celui du ou des tubes (15) d'injection de liquide (12) et l'extrémité ouverte de sortie du tube (13) du dispositif d'injection de gaz (3) est positionnée à l'intérieur du tube d'injection de liquide de manière coaxiale.

De préférence, le tube d'injection de liquide (15) de liquide comprend un déflecteur.

De préférence, l'extrémité du tube d'injection de liquide (15) présente un rétrécissement du diamètre de sortie.

De préférence, le réacteur comprend en outre
- une enceinte réactionnelle, de forme allongée le long d'un axe vertical, pouvant contenir
   une phase liquide située dans une zone inférieure comprenant, et de préférence constituée, des produits de la réaction, de la charge oléfinique gazeuse et dissoute, de préférence de l'éthylène dissout et gazeux, d'un système catalytique et d'un éventuel solvant, et une phase gazeuse située dans une zone supérieure au-dessus de la zone inférieure comprenant de la charge oléfinique gazeuse et de préférence de l'éthylène gazeux, ainsi que des gaz incondensables (éthane notamment),
- un moyen d'introduction du système catalytique, ledit moyen étant situé dans la partie inférieure de l'enceinte réactionnelle,
- une boucle de recirculation comprenant un moyen de soutirage à la base (de préférence au fond) de l'enceinte réactionnelle pour le soutirage d'une fraction liquide, un échangeur thermique permettant le refroidissement dudit liquide, et un moyen d'introduction dudit liquide refroidi, ledit moyen d'introduction étant situé dans la partie supérieure de la zone inférieure de l'enceinte réactionnelle,
- et éventuellement une boucle de recycle de la phase gazeuse pour recycler au moins une fraction de la phase gazeuse vers la zone inférieure de la phase liquide, comprenant un moyen de soutirage situé dans la zone supérieure de l'enceinte réactionnelle pour permettre le soutirage d'une fraction gazeuse au niveau de la phase gazeuse et un moyen d'introduction positionné dans la zone inférieure de l'enceinte réactionnelle pour permettre d'introduire ladite fraction gazeuse soutirée dans la phase liquide.

L' objet de la présente invention concerne un procédé d'oligomérisation d'une charge oléfinique gazeuse mettant en œuvre un réacteur gaz/liquide tel que défini précédemment, ledit procédé comprenant la mise en contact d'un liquide comprenant un système catalytique comprenant un catalyseur métallique, au moins un activateur et au moins un additif, et éventuellement un solvant, et de la charge oléfinique gazeuse au moyen d'un dispositif d'injection de gaz et d'un dispositif d'injection de liquide, lesdits dispositifs d'injection étant agencés de manière à ce que l'injection du liquide entraine une diminution par cisaillement de la taille des bulles d'éthylène gazeux.

De préférence, la charge oléfinique gazeuse comprend entre 2 et 6 atomes de carbone, de préférence entre 2 et 4 atomes de carbone.

De préférence, la vitesse d'injection du liquide est supérieure à la vitesse d'injection de la charge oléfinique gazeuse de manière à favoriser le cisaillement de la taille des bulles d'oléfines gazeuses en bulles gazeuses de taille plus petite.

### DEFINITIONS & ABREVIATIONS

Dans l'ensemble de la description, les termes ou abréviations ci-après ont le sens suivant. On entend par oligomérisation d'oléfines toute réaction d'addition d'une première oléfine sur une seconde oléfine, identique ou différente de la première. L'oléfine ainsi obtenue a pour formule brute CₙH₂ₙ où n est égal ou supérieur à 4.

On entend par alpha-oléfine linéaire une oléfine sur laquelle la double liaison est située en position terminale de la chaine alkyle linéaire.

On entend par système catalytique une espèce chimique qui permet la mise en œuvre du catalyseur. Le système catalytique peut être un précurseur métallique comprenant un ou plusieurs atomes métalliques ou un mélange de composés permettant de catalyser une réaction chimique, et plus spécifiquement une réaction d'oligomérisation d'oléfines. Le mélange de composés comprend au moins un précurseur métallique. Le mélange de composés peut en outre comprendre un agent activateur. Le mélange de composés peut comprendre un additif. Le composé ou le mélange de composés peuvent optionnellement être en présence d'un solvant.

On entend par phase liquide, le mélange de l'ensemble des composés qui se trouvent à un état physique liquide dans les conditions de température et de pression de l'enceinte réactionnelle.

On entend par phase gazeuse le mélange de l'ensemble des composés qui se trouvent à l'état physique gaz dans les conditions de température et de pression de l'enceinte réactionnelle : sous forme de bulles présentes dans le liquide, et également dans la partie haute du réacteur (ciel gazeux du réacteur).

On entend par zone inférieure de l'enceinte réactionnelle, la partie de l'enceinte comprenant la phase liquide, la charge oléfinique gazeuse, en particulier de l'éthylène gazeux, avantageusement sous forme de bulles ou dissoute, les produits de la réaction tels que l'alpha oléfine linéaire souhaitée (i.e. butène-1, hexène-1, octène-1 ou le mélange d'alpha-oléfines linéaires), le système catalytique et optionnellement un solvant. De préférence, la zone inférieure de l'enceinte réactionnelle représente au moins la moitié, de préférence les trois-quarts du volume total de l'enceinte réactionnelle.

On entend par zone supérieure de l'enceinte réactionnelle, la partie de l'enceinte se situant au sommet de l'enceinte, c'est-à-dire directement au-dessus de la zone inférieure et constituée de phase gazeuse correspondant au ciel gazeux.

On entend par gaz incondensable, une espèce sous forme physique gaz qui ne se dissout que partiellement dans le liquide aux conditions de température et de pression de l'enceinte réactionnelle, et qui peut, dans certaines conditions, s'accumuler dans le ciel du réacteur (par exemple ici : l'éthane).

On désigne par les termes réacteur ou dispositif, l'ensemble des moyens permettant la mise en œuvre du procédé d'oligomérisation selon l'invention, tel que notamment l'enceinte réactionnelle et la boucle de recirculation.

On entend par partie inférieure de l'enceinte réactionnelle, le quart inférieur de l'enceinte réactionnelle, dans la zone inférieure contenant la phase liquide.

On entend par partie supérieure de l'enceinte réactionnelle, le quart supérieur de l'enceinte réactionnelle et appartenant à la zone inférieure qui est destinée à contenir la phase liquide.

L'expression taux de saturation en charge oléfinique gazeuse dissout, en particulier en éthylène dissout, désigne le ratio de la quantité charge oléfinique gazeuse dissoute, en particulier d'éthylène dissout, sur la quantité maximale de la charge oléfinique gazeuse dissout, en particulier d'éthylène, qu'il est possible de dissoudre dans le liquide dans les conditions de température et de pression considérées.

Les différents composants du réacteur vont être décrits en référence à l'ensemble des figures, chaque composant conservant la même référence d'une figure à l'autre.

### DESCRIPTION SUCCINTE DES FIGURES

La figure 1 illustre un réacteur selon l'art antérieur. Ce réacteur est constitué d'une enceinte réactionnelle 1 comprenant une zone inférieure comprenant une phase liquide et une zone supérieure comprenant une phase gazeuse, d'un moyen d'introduction de la charge oléfinique gazeuse 2, en particulier de l'éthylène gazeux, par l'intermédiaire d'un dispositif d'injection gazeux 3 dans la phase liquide. La phase gazeuse dans la partie supérieure de l'enceinte réactionnelle 1 comprend un moyen de purge 4. Dans le fond de l'enceinte réactionnelle 1 se situe une conduite pour le soutirage d'une fraction liquide 5. Ladite fraction 5 est divisée en 2 flux, un premier flux principal 7 envoyé vers un échangeur à chaleur 8 puis introduit par l'intermédiaire d'une conduite 9 dans la phase liquide et un second flux 6 correspondant à l'effluent envoyé vers une étape ultérieure. La conduite 10 dans le fond de l'enceinte réactionnelle permet l'introduction du système catalytique.

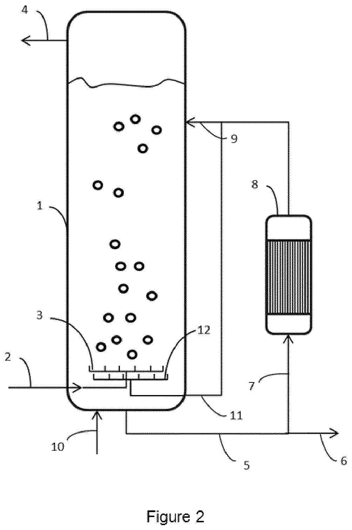

La Figure 2 illustre un réacteur selon l'invention. Ce réacteur diffère de celui de la Figure 1 en ce que le flux en sortie de l'échangeur à chaleur 8 est divisé en deux flux 9 et 11, et que le flux 11 est envoyé dans la zone liquide de la même enceinte 1 par l'intermédiaire d'un dispositif d'injection liquide 12 agencé avec le dispositif d'injection gazeux 3 de manière à ce que l'injection du liquide puisse entrainer par cisaillement une diminution de la taille des bulles d'éthylène lors de l'introduction de l'éthylène gazeux et du flux 11.

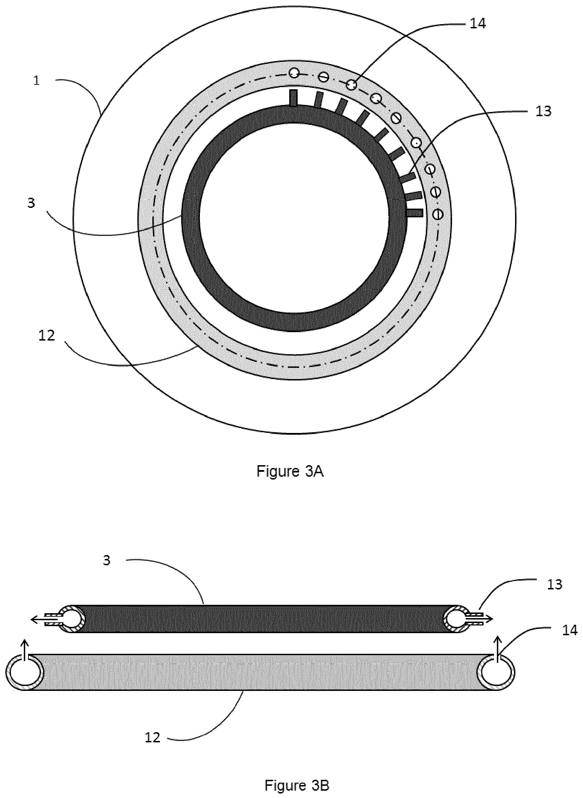

La Figure 3A est une vue schématique selon l'axe vertical de l'enceinte (pouvant être pardessus ou par dessous) d'un mode de réalisation des dispositifs d'injection de gaz 3 et de liquide 12 selon l'invention disposés dans une enceinte réactionnelle 1. Le dispositif d'injection de gaz 3 et de liquide 12 sont de forme annulaire et agencés de manière à ce que les orifices de sortie de gaz prolongés chacun par un tube 13 du dispositif d'injection de gaz 3 injectent le gaz vers la paroi extérieure de l'enceinte 1 et que la trajectoire d'injection du gaz croise perpendiculairement la trajectoire de l'orifice 14 d'injection du liquide de manière à provoquer le cisaillement du gaz afin de diminuer la taille des bulles de gaz injecté.
La figure 3B est une vue schématique d'une coupe selon l'axe verticale du dispositif d'injection de la figure 3A. Le dispositif d'injection de liquide 12 est un anneau circulaire ayant un diamètre supérieur à celui du dispositif d'injection de gaz 3, lui-même sous forme d'anneau circulaire. Le dispositif d'injection de liquide 12 est positionné sur un plan différent de celui du dispositif d'injection de gaz 3. Sur la figure 3B le dispositif de liquide 12 est sur un plan inférieur à celui du dispositif d'injection de gaz 3, et de sorte que les orifices d'injection de gaz 3 soit positionnés sur la trajectoire des orifices 14 du dispositif d'injection de liquide 12.

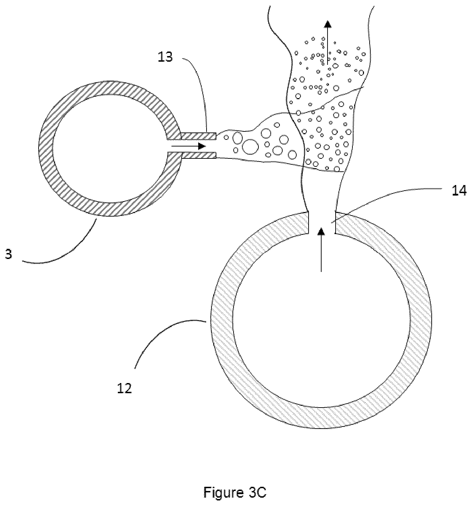

La figure 3C est une vue schématique d'une coupe selon l'axe vertical des dispositifs d'injection selon la figure 3A illustrant l'effet de cisaillement du flux gazeux injecté par le dispositif d'injection de gaz 3 par le flux de liquide injecté par le dispositif d'injection de liquide 12.

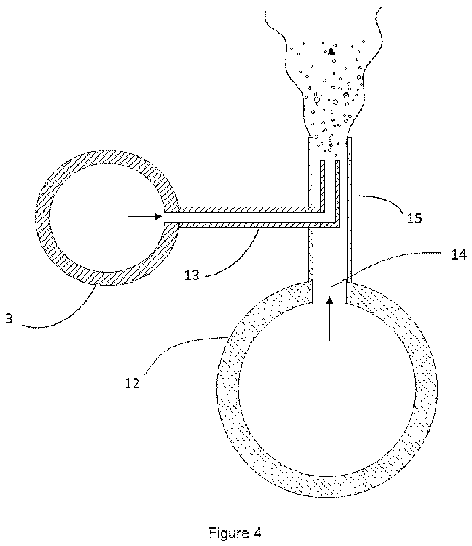

La Figure 4 est une vue schématique d'une coupe selon l'axe vertical illustrant une variante du mode d'agencement des dispositifs d'injection de gaz 3 et de liquide 12. Dans cette variante, l'orifice d'injection de gaz est prolongé par un tube 13, de même que l'orifice d'injection de liquide 14 est prolongé par un tube 15. Le tube d'injection de gaz 13 présente une courbure à 90°, et l'extrémité ouverte du tube 13 se positionne de manière concentrique à l'intérieur du tube 15 prolongeant l'orifice 14 d'injection du dispositif d'injection de liquide 12. L'extrémité ouverte du tube 13 d'injection de gaz, au niveau de laquelle se trouve la sortie du gaz est positionnée au centre du tube d'injection de liquide. Ainsi, les flux d'injection gaz et liquide sont orientés dans la même direction, et le cisaillement du gaz par le liquide est obtenu par l'enrobement et l'arrachage du gaz par le liquide.

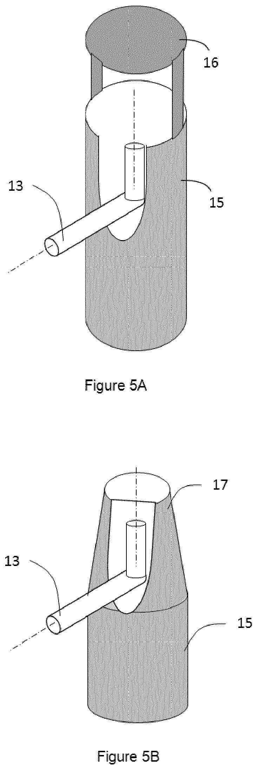

La Figure 5A illustre une variante du mode de réalisation de la figure 4 consistant à positionner un déflecteur 16 à l'extrémité du tube d'injection de liquide 15. L'obstruction de l'écoulement gaz-liquide en sortie du dispositif entrainé par le déflecteur permet d'améliorer le cisaillement des bulles de gaz par la génération d'une turbulence additionnelle qui permet d'améliorer la brisure des bulles de gaz en bulles gazeuses de taille plus petite.
La Figure 5B illustre une variante du mode de réalisation de la figure 4 consistant en un rétrécissement 17 à l'extrémité du tube d'injection du liquide 15. Ce rétrécissement entraine l'accélération du mélange gaz-liquide lors de l'injection dudit mélange ce qui permet d'augmenter le cisaillement et ainsi favoriser la brisure des bulles de gaz en bulles gazeuses de taille plus petite.

### DESCRIPTION DETAILLEE DE L'INVENTION

Il est précisé que, dans toute cette description, l'expression « compris(e) entre ... et ... » doit s'entendre comme incluant les bornes citées.

Dans le sens de la présente invention, les différents modes de réalisation présentés peuvent être utilisés seuls ou en combinaison les uns avec les autres, sans limitation de combinaison.

Dans le sens de la présente invention, les différentes plages de paramètres pour une étape donnée telles que les plages de pression et les plages de température peuvent être utilisées seules ou en combinaison. Par exemple, dans le sens de la présente invention, une plage de valeur préférée de pression peut être combinée avec une plage de valeur de température plus préférée.

Dans la suite de la description et dans les revendications, les positions (« fond », « sommet », « au-dessus », « en-dessous », « horizontal », « vertical », « moitié inférieure », etc.) des différents éléments sont définies par rapport à la colonne en position de fonctionnement.

La présente invention concerne un procédé utilisant un réacteur gaz/liquide d'oligomérisation d'une charge oléfinique gazeuse comprenant un dispositif d'injection de gaz et un dispositif d'injection de liquide, lesdits dispositifs d'injection étant agencés de manière à ce que l'injection du liquide puisse entrainer une diminution par cisaillement de la taille des bulles de la charge oléfinique gazeuse lors de l'injection de ladite charge.

Dans le sens de la présente invention, le dispositif d'injection de gaz est destiné à injecter dans un réacteur d'oligomérisation une charge oléfinique à l'état gazeux. Avantageusement, le réacteur selon l'invention permet d'améliorer la dissolution des oléfines gazeuses dans la phase liquide qi contient le système catalytique et de réduire la vitesse d'ascension des oléfines gazeuses dans la phase liquide, ce qui diminue de manière synergique le phénomène de perçage. En effet, plus les bulles d'oléfines gazeuse injectées sont petites, plus leur vitesse d'ascension dans la phase liquide est faible.

Ainsi, avantageusement le taux de saturation en oléfines gazeuses dissoutes, de préférence en éthylène gazeux dissout, dans la phase liquide est supérieur à 70,0 %, de préférence entre 70,0 et 100 %, de préférence entre 80,0 et 100 %, de manière préférée compris entre 80,0 et 99,0 %, de préférence entre 85,0 et 99,0 % et de manière encore plus préférée entre 90,0 et 98,0 %.

Le taux de saturation en oléfines gazeuse dissoute, de préférence en éthylène gazeux dissout, peut être mesuré par toute méthode connue de l'Homme du métier et par exemple par l'analyse chromatographique en phase gaz (couramment appelée GC) d'une fraction de la phase liquide soutirée de l'enceinte réactionnelle.

Un autre avantage de la présente invention est d'améliorer la conversion de la charge oléfinique, en particulier d'éthylène, et/ou la sélectivité en oléfines, en particulier en alpha oléfines, ainsi que la productivité volumique du procédé d'oligomérisation.

Ainsi, un autre avantage du réacteur selon l'invention est de permettre de réduire le volume réactionnel et donc les dimensions du réacteur à performances identiques par rapport à un réacteur selon l'art antérieur.

### REACTEUR

Le procédé de la présente invention concerne un réacteur gaz/liquide d'oligomérisation d'une charge oléfinique gazeuse comprenant un dispositif d'injection de gaz et un dispositif d'injection de liquide, lesdits dispositifs d'injection étant agencés de manière à ce que l'injection du liquide puisse entrainer une diminution par cisaillement de la taille des bulles lors de l'injection de la charge oléfinique gazeuse.

L'effet de cisaillement du flux gaz par le flux liquide permet de briser les bulles de gaz, et ainsi réduire leur taille et donc améliorer leur dissolution dans la phase liquide. Ainsi, l'agencement des dispositifs d'injection de gaz et de liquide selon l'invention permet de diminuer la taille des bulles de gaz, de préférence d'éthylène gazeux, d'accélérer la dissolution du gaz, de préférence de l'éthylène gazeux, dans la phase liquide.

De préférence, le réacteur gaz/liquide d'oligomérisation est un réacteur gaz/liquide de dimérisation, trimérisation ou tétramérisation de l'éthylène.

Le dispositif d'injection de gaz 3 comprend au moins un orifice d'injection de gaz et le dispositif d'injection de liquide (12) comprend au moins un orifice d'injection de liquide, chaque orifice d'injection de gaz étant situé à proximité d'un orifice du dispositif d'injection de liquide 11 et positionné de manière à ce que la trajectoire d'injection du gaz soit dans le plan de la trajectoire d'injection du liquide. L'injection du liquide peut alors provoquer le cisaillement du gaz injecté et entrainer la diminution de la taille des bulles de gaz permettant d'améliorer la dissolution du gaz dans la phase liquide via une augmentation de l'interface entre le gaz et le liquide.

Il est entendu qu'au sens de la présente invention les dispositifs d'injection de gaz et de liquide peuvent comprendre une pluralité d'orifices d'injection respectivement de gaz et de liquide en fonction des dimensions du réacteur.

Avantageusement, le réacteur selon l'invention, avec l'agencement particulier des dispositifs d'injection de gaz et de liquide, permet de diminuer la taille des bulles de gaz injectées d'au moins 20% par rapport à la taille des bulles de gaz injectées sans cisaillement. De préférence, le pourcentage de diminution de la taille des bulles de gaz par cisaillement est au moins de 25% par rapport à la taille des bulles de gaz injectées sans cisaillement, de préférence d'au moins 30%, de préférence d'au moins 35% et de manière préférée d'au moins 40%.

Avantageusement, la brisure d'une bulle de gaz en deux plus petites de même taille génère une augmentation de surface d'échange entre le gaz et le liquide de 26%, une brisure d'une bulle de gaz en 4 bulles plus petites de même taille génère une augmentation de 59%, une brisure d'une bulle de gaz en 6 bulles plus petites de même taille génère une augmentation de 82%. Ainsi, un réacteur selon l'invention facilite et améliore donc de manière significative l'absorption de gaz dans la phase liquide ce qui permet d'augmenter la saturation en oléfines gazeuses dans la phase liquide et de limiter le phénomène de perçage.

De préférence, le dispositif d'injection de gaz 3 est choisi parmi une conduite, un réseau de conduites, un distributeur multitubulaire, une plaque perforée, un tube cylindrique, un tube concentrique ou tout autre moyen connu de l'Homme du métier.

On entend par orifice d'injection, un trou rond, un trou ovale, une fente ou toutes autres formes permettant l'injection du liquide ou du gaz dans le réacteur.

De préférence, les orifices d'injection de gaz sont circulaires, c'est-à-dire des trous ronds. De préférence, les orifices d'injection de gaz présentent un diamètre compris entre 1,0 et 20,0 mm, de préférence entre 3,0 et 15,0 mm, pour former des bulles d'éthylène dans le liquide de dimension millimétrique.

De préférence, les orifices d'injection de liquide présentent un diamètre compris entre 1,0 et 15,0 mm, de préférence entre 3,0 et 20,0 mm.

De préférence, les orifices d'injection du liquide sont circulaires, c'est-à-dire des trous ronds. De préférence, les orifices d'injection de gaz et de liquide sont circulaires et les orifices d'injection de liquide présentent un diamètre supérieur ou égal au diamètre des orifices d'injection de gaz. De préférence, le rapport entre le diamètre d'un orifice d'injection de gaz et le diamètre de l'orifice d'injection de liquide agencé à proximité dudit orifice d'injection de gaz est compris entre 0,1 et 1,0, de préférence entre 0,4 et 0,8.

Dans un mode de réalisation préféré, les orifices des dispositifs d'injection de gaz et/ou de liquide sont prolongés par un tube. De préférence, les orifices des dispositifs d'injection de gaz et de liquide sont prolongés par un tube et les tubes d'injection de gaz 13 du dispositif d'injection de gaz présentent un diamètre inférieur à celui des tubes d'injection de liquide 15, de manière préférée l'extrémité ouverte de sortie de chaque tube d'injection de gaz 13 étant positionnée à l'intérieur d'un tube d'injection de liquide de manière coaxiale. L'orifice de sortie du tube d'injection de gaz est dirigé vers l'orifice de sortie du tube d'injection de liquide.

De préférence, le tube d'injection de liquide 15 comprend un déflecteur comme moyen d'obturation partielle du tube, de préférence une plaque circulaire ou carrée, perforée ou non, comme illustré à la figure 5A. Avantageusement, le déflecteur permet d'améliorer l'effet de cisaillement des bulles de gaz par le liquide.

De préférence, l'extrémité de sortie du tube d'injection de liquide présente un rétrécissement du diamètre de sortie, comme illustré à la figure 5B. Ledit rétrécissement entraine l'accélération du mélange gaz-liquide ce qui permet d'augmenter les forces de cisaillement et améliore encore la brisure des bulles de gaz en bulles gazeuses de taille plus petite.

Dans un mode très préféré, le tube présente un rétrécissement du diamètre de sortie et un déflecteur.

De préférence, les dispositifs d'injection de gaz, de préférence de l'éthylène gazeux, et du liquide sont positionnés dans l'enceinte réactionnelle, de préférence dans la partie inférieure.

Avantageusement, un orifice d'injection de gaz et un orifice d'injection de liquide sont positionnés en regard l'un par rapport à l'autre selon un angle compris entre 0° et 180°. Lorsque les orifices des dispositifs d'injection de gaz et de liquide sont prolongés par un tube, les orifices d'injection de gaz et de liquide correspondent aux orifices de sorties du ou des tubes d'injection de gaz et de liquide. Un angle de 0° signifie que le gaz et le liquide sont injectés par lesdits orifices d'injection respectifs sur le même axe de trajectoire et dans le même sens, comme illustré à la figure 4. De préférence, l'angle formé par les trajectoires est compris entre 0° et 120°, de préférence entre 30° et 120°, de préférence entre 45° et 90°. De manière très préférée, l'angle formé par les trajectoires est compris entre 0° et 90°. De préférence, l'angle formé par les trajectoires est égal à 0°, 30°, 45°, 90°, 120° ou 180°.

Dans un mode de réalisation particulier, le dispositif d'injection de gaz est un tube cylindrique ayant une forme d'anneau circulaire par exemple rond ou ovale, et présentant des orifices d'injection. Avantageusement, le dispositif d'injection de liquide est également un tube cylindrique ayant une forme d'anneau sensiblement circulaire par exemple rond ou ovale, et présentant des orifices d'injection. Conformément à l'invention, ledit dispositif d'injection de liquide est positionné à proximité dudit dispositif d'injection de gaz et de sorte qu'un (ou chaque) orifice d'injection de gaz soit positionné à proximité d'un orifice du dispositif d'injection de liquide 11 de manière à ce que la trajectoire d'injection de liquide soit dans le même plan de la trajectoire d'injection du gaz afin de provoquer le cisaillement dudit gaz.

Très avantageusement, le dispositif d'injection de gaz est sous forme d'anneau, de préférence circulaire, et présente un diamètre supérieur ou inférieur à celui du dispositif d'injection de liquide sous forme d'anneau, de préférence circulaire. Lorsque le diamètre du dispositif d'injection de gaz est inférieur à celui du dispositif d'injection de liquide, le dispositif d'injection de gaz se positionne à l'intérieur du dispositif d'injection de liquide sur un plan différent, c'est-à-dire supérieur ou inférieur, comme illustré à la figure 3A. Inversement, lorsque le diamètre du dispositif d'injection de gaz est supérieur à celui du dispositif d'injection de liquide, le dispositif d'injection de gaz se positionne à l'extérieur du dispositif d'injection de liquide sur un plan différent, c'est-à-dire supérieur ou inférieur.

Dans un mode de réalisation particulier, un enchainement de plusieurs dispositifs d'injection de liquide et de gaz sous forme d'anneau circulaire de diamètres décroissants sont alternés **de la périphérie** vers le centre représenté par l'axe central du dispositif ayant le diamètre le plus grand. Lesdits dispositifs sont positionnés les uns par rapport aux autres de sorte qu'un orifice d'injection de gaz d'un dispositif d'injection de gaz soit positionné à proximité d'un orifice du dispositif d'injection de liquide adjacent de manière à ce que la trajectoire d'injection de liquide soit dans le même plan de la trajectoire d'injection du gaz afin de provoquer le cisaillement dudit gaz.

En particulier, le réacteur gaz/liquide peut comprendre en outre :
- une enceinte réactionnelle 1, de forme allongée le long de l'axe vertical central, pouvant contenir
   une phase liquide située dans une zone inférieure comprenant, et de préférence constituée, des produits de la réaction, de la charge oléfinique gazeuse et dissoute, de préférence de l'éthylène dissout et gazeux, d'un système catalytique et d'un éventuel solvant, et une phase gazeuse située dans une zone supérieure au-dessus de la zone inférieure comprenant de l'éthylène gazeux, ainsi que les gaz incondensables (éthane notamment),
- un moyen d'introduction du système catalytique, optionnellement ledit moyen étant situé dans la partie inférieure de l'enceinte réactionnelle,
- une boucle de recirculation comprenant un moyen de soutirage à la base (de préférence au fond) de l'enceinte réactionnelle pour le soutirage d'une fraction liquide, un échangeur thermique permettant le refroidissement dudit liquide, et un moyen d'introduction dudit liquide refroidi, ledit moyen d'introduction étant situé dans la partie supérieure de la zone inférieure de l'enceinte réactionnelle,
- et éventuellement une boucle de recycle de la phase gazeuse pour recycler au moins une fraction de la phase gazeuse vers la zone inférieure de la phase liquide, comprenant un moyen de soutirage situé dans la zone supérieure de l'enceinte réactionnelle pour permettre le soutirage d'une fraction gazeuse au niveau de la phase gazeuse et un moyen d'introduction positionné dans la zone inférieure de l'enceinte réactionnelle pour permettre d'introduire ladite fraction gazeuse soutirée dans la phase liquide.

De préférence, l'enceinte de réactionnelle 1 est cylindrique. Dans le cas d'une enceinte cylindrique, le diamètre D est le diamètre du cylindre. Une telle géométrie permet notamment de limiter la présence de volumes « morts » dans la colonne.

### PROCEDE D'OLIGOMERISATION

L' objet de l'invention concerne le procédé d'oligomérisation d'une charge oléfinique gazeuse, de préférence d'éthylène gazeux, mettant en œuvre un réacteur gaz/liquide selon l'invention tel que défini ci-dessus, ledit procédé comprenant la mise en contact d'un liquide et de la charge oléfinique gazeuse, de préférence d'éthylène gazeux, au moyen d'un dispositif d'injection de gaz et d'un dispositif d'injection de liquide, lesdits dispositifs d'injection étant agencés de manière à ce que l'injection du liquide entraine une diminution par cisaillement de la taille des bulles d'éthylène gazeux.

Dans un fluide en mouvement, toute différence de vitesse au sein d'un fluide entraîne des contraintes de cisaillement : les particules fluides allant plus vite sont freinées par celles allant moins vite. La mise en contact d'une phase gaz avec une phase liquide qui a une vitesse d'injection supérieure à celle du gaz entraine des contraintes de cisaillement à l'interface gaz/liquide provoquant la brisure des bulles de gaz. Le cisaillement est une résultante de la turbulence, et peut être mesuré indirectement par des mesures de fluctuation de vitesse de chaque phase ou calculé par les méthodes connues par l'homme de l'art.

De préférence, la vitesse d'injection du liquide est supérieure à la vitesse d'injection de la charge oléfinique gazeuse de manière à favoriser le cisaillement des bulles de gaz et la réduction de la taille des bulles d'oléfines gazeuses en bulles gazeuses de taille plus petite.

De préférence, la vitesse d'injection du liquide est comprise entre 0,1 et 20 m/s (mètre/seconde) pour le liquide, la vitesse d'injection du gaz est comprise entre 1,0 et 10 m/s. Le procédé d'oligomérisation d'une charge oléfinique gazeuse mettant en œuvre le réacteur selon l'invention permet de produire des alpha oléfines linéaires par la mise en contact de ladite charge oléfinique et d'un système catalytique, éventuellement en présence d'un solvant. La charge oléfinique gazeuse comprend de préférence entre 2 et 6 atomes de carbone, de préférence entre 2 et 4 atomes de carbone. De préférence, la charge oléfinique est choisie parmi le butène, plus particulièrement l'isobutène ou le butène-1, le propylène, et l'éthylène, seul ou en mélange.

Tous les systèmes catalytiques connus de l'Homme du métier et aptes à être mis en œuvre dans les procédés de dimérisation, de trimérisation, de tétramérisation et plus généralement dans les procédés d'oligomérisation selon l'invention, font partie du domaine de l'invention.

Lesdits systèmes catalytiques ainsi que leur mise en oeuvre sont notamment décrits dans les demandes FR2984311, FR2552079, FR3019064, FR3023183, FR3042989 ou encore dans la demande FR3045414.

De préférence, les systèmes catalytiques comprennent, de préférence sont constitués de :
- un précurseur métallique de préférence à base de nickel, de titane ou de chrome,
- optionnellement un agent activateur,
- optionnellement un additif, et
- optionnellement un solvant.

### Le précurseur métallique

Le précurseur métallique utilisé dans le système catalytique est choisi parmi les composés à base de nickel, de titane ou de chrome.

Dans un mode de réalisation, le précurseur métallique est à base de nickel et préférentiellement comprend du nickel de degré d'oxydation (+II). De préférence, le précurseur de nickel est choisi parmi les carboxylates de nickel(II) tel que par exemple le 2-éthylhexanoate de nickel, les phénates de nickel(II), les naphténates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de π-allylnickel(II), le bromure de π-allylnickel(Il), le dimère du chlorure de methallylnickel(II), l'hexafluorophosphate de η³-allylnickel(Il), l'hexafluorophosphate de η³-methallylnickel(II) et le 1,5-cyclooctadiényle de nickel(II), sous leur forme hydratée ou non, pris seul ou en mélange.

Dans un second mode de réalisation, le précurseur métallique est à base de titane et préférentiellement comprend un composé aryloxy ou alcoxy du titane.

Le composé alcoxy du titane répond avantageusement à la formule générale [Ti(OR)₄] dans laquelle R est un radical alkyle linéaire ou ramifié. Parmi les radicaux alcoxy préférés, on peut citer à titre d'exemple non limitatifs : le tétraéthoxy, le tétraisopropoxy, le tétra-n-butoxy et le tétra-2-éthyl-hexyloxy.

Le composé aryloxy du titane répond avantageusement à la formule générale [Ti(OR')₄] dans laquelle R' est un radical aryle substitué ou non par des groupements alkyle ou aryle. Le radical R' peut comporter des substituants à base d'hétéroatome. Les radicaux aryloxy préférés sont choisis parmi le phénoxy, le 2-méthylphénoxy, le 2,6-diméthylphénoxy, le 2,4,6-triméthylphénoxy, le 4-méthylphénoxy, le 2-phénylphénoxy, le 2,6-diphénylphénoxy, le 2,4,6-triphénylphénoxy, le 4-phénylphénoxy, le 2-tert-butyl-6-phénylphénoxy, le 2,4-ditertbutyl-6-phénylphénoxy, le 2,6-diisopropylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy, le radical biphénoxy, le binaphtoxy, le 1,8-naphtalène-dioxy.

Selon un troisième mode de réalisation, le précurseur métallique est à base de chrome et préférentiellement comprend un sel de chrome (II), un sel de chrome (III), ou un sel de degré d'oxydation différent pouvant comporter un ou plusieurs anions identiques ou différents, tels que par exemple des halogénures, des carboxylates, des acétylacétonates, des anions alcoxy ou aryloxy. De préférence, le précurseur à base de chrome est choisi parmi CrCl₃, CrCl₃(tétrahydrofurane)₃, Cr(acétylacétonate)₃, Cr(naphténate)₃, Cr(2-éthylhexanoate)₃, Cr(acétate)₃.

La concentration en nickel, en titane ou en chrome, est comprise entre 0,001 et 300,0 ppm en masse de métal atomique par rapport à la masse réactionnelle, de préférence entre 0,002 et 100,0 ppm, préférentiellement entre 0,003 et 50,0 ppm, plus préférentiellement entre 0,05 et 20,0 ppm et encore plus préférentiellement entre 0,1 et 10,0 ppm en masse de métal atomique par rapport à la masse réactionnelle.

### L'agent activateur

Optionnellement, quel que soit le précurseur métallique, le système catalytique comprend un ou plusieurs agents activateurs choisis parmi les composés à base d'aluminium tels que le dichlorure de méthylaluminium (MeAlCl₂), le dichloroéthylaluminium (EtAlCl₂), le sesquichlorure d'éthylaluminium (Et₃Al₂Cl₃), le chlorodiéthylaluminium (Et₂AlCl), le chlorodiisobutylaluminium (i-Bu₂AlCl), le triéthylaluminium (AlEt₃), le tripropylaluminium (Al(n-Pr)₃), le triisobutylaluminium (Al(i-Bu)₃), le diéthyl-éthoxyaluminium (Et₂AlOEt), le méthylaluminoxane (MAO), l'éthylaluminoxane et les méthylaluminoxanes modifiés (MMAO).

### L'additif

Optionnellement, le système catalytique comprend un ou plusieurs additifs.

L'additif est choisi parmi les composés phosphorés monodentés, des composés phosphorés bidentés, des composés phosphorés tridentés, des composés oléfiniques, des composés aromatiques, des composés azotés, des bipyridines, des diimines, des éthers monodentés, des éthers bidentés, des thioéthers monodentés, des thioéthers bidentés, des carbènes monodentés ou bidentés, des ligands mixtes tels que des phosphinopyridines, des iminopyridines, des bis(imino)pyridines

Lorsque le système catalytique est à base de nickel, l'additif est choisi parmi :
- les composés de type azoté, tels que la triméthylamine, la triéthylamine, le pyrrole, le 2,5-diméthylpyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, ou
- les composés de type phosphine choisi indépendamment parmi la tributylphosphine, la triisopropylphosphine, la tricyclopentylphosphine, la tricyclohexylphosphine, la triphénylphosphine, la tris(o-tolyl)phosphine, le bis(diphénylphosphino)éthane, l'oxyde de trioctylphosphine, l'oxyde de triphénylphosphine, la triphénylphosphite, ou
- les composés répondant à la formule générale (I) ou un des tautomères dudit composé : 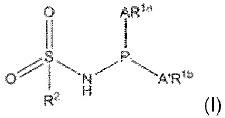 dans laquelle
   - A et A', identiques ou différents, sont indépendamment un oxygène ou une liaison simple entre l'atome de phosphore et un atome de carbone,
   - les groupements R^{1a} et R^{1b} sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 2-méthylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle,
   - le groupement R² est choisi indépendamment parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle.

Lorsque le système catalytique est à base de titane, l'additif est choisi parmi l'éther diéthylique, le diisopropyléther, le dibutyléther, le diphényléther, le 2-méthoxy-2-méthylpropane, 2-methoxy-2-méthylbutane, le diméthoxy-2,2 propane, le di(2-éthylhexyloxy)-2,2 propane, le 2,5-dihydrofurane, le tétrahydrofurane, le 2-méthoxytétrahydrofurane, le 2-méthyltétrahydrofurane, le 3-méthyltétrahydrofurane, le 2,3-dihydropyrane, le tétrahydropyrane, le 1,3-dioxolane, le 1,3-dioxane, le 1,4-dioxane, le diméthoxyéthane, di(2-méthoxyéthyl)éther, le benzofurane, le glyme et le diglyme pris seuls ou en mélange.

Lorsque le système catalytique est à base de chrome, l'additif est choisi parmi :
- les composés de type azoté, tels que la triméthylamine, la triéthylamine, le pyrrole, le 2,5-diméthylpyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, ou
- les composés aryloxy de formule générale [M(R³O)₂₋ₙXₙ]_{y} dans laquelle
   * M est choisi parmi le magnésium, le calcium, le strontium et le baryum, de préférence le magnésium,
   * R³ est un radical aryl contenant de 6 à 30 atomes de carbone, X est un halogène ou un radical alkyl contenant de 1 à 20 atomes de carbone,
   * n est un nombre entier qui peut prendre les valeurs de 0 ou 1, et
   * y est un nombre entier compris entre 1 et 10, de préférence y est égal à 1, 2, 3 ou 4.

De préférence, le radical aryloxy R³O est choisi parmi le 4-phénylphénoxy, le 2-phénylphénoxy, le 2,6-diphénylphénoxy, le 2,4,6-triphénylphénoxy, le 2,3,5,6-tétraphénylphénoxy, le 2-tert-butyl-6-phénylphénoxy, le 2,4-ditertbutyl-6-phénylphénoxy, le 2,6-diisopropylphénoxy, le 2,6-diméthylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy. Les deux radicaux aryloxy peuvent être portés par une même molécule, comme par exemple le radical biphénoxy, le binaphtoxy ou le 1,8-naphtalène-dioxy, De préférence, le radical aryloxy R³O est le 2,6-diphénylphénoxy, le 2-tert-butyl-6-phénylphénoxy ou le 2,4-ditert-butyl-6-phénylphénoxy.

### Le solvant

Dans un autre mode de réalisation selon l'invention, le système catalytique comprend optionnellement un ou plusieurs solvants.

Dans un mode de réalisation, un solvant ou un mélange de solvants peut être utilisé durant la réaction d'oligomérisation.

Le ou les solvants sont avantageusement choisis parmi les éthers, les alcools, les solvants halogénés et les hydrocarbures, saturés ou insaturés, cycliques ou non, aromatiques ou non, comprenant entre 1 et 20 atomes de carbone, de préférence entre 4 et 15 atomes de carbone, préférentiellement entre 4 et 12 atomes de carbone et encore plus préférentiellement entre 4 et 8 atomes de carbone.

De préférence, le solvant est choisi parmi le pentane, l'hexane, le cyclohexane, le méthylcyclohexane, l'heptane, le butane ou l'isobutane, le cycloocta-1,5-diène, le benzène, le toluène, l'ortho-xylène, le mésitylène, l'éthylbenzène, le diéthyléther, le tétrahydrofurane, le 1,4-dioxane, le dichlorométhane, le dichloroéthane, le tétrachloroéthane, l'hexachloroéthane, le chlorobenzène, le dichlorobenzène, le butène, l'hexène et l'octène purs ou en mélange.

De préférence, le solvant peut être avantageusement choisi parmi les produits de la réaction d'oligomérisation. De manière préférée, le solvant utilisé est le cyclohexane.

De préférence, lorsqu'un solvant est mis en œuvre dans le procédé d'oligomérisation, le taux massique de solvant introduit le réacteur mis en œuvre dans le procédé selon l'invention est compris entre 0,2 et 10,0, de préférence entre 0,5 et 5,0, et de manière préférée entre 1,0 et 4,0. Le taux de solvant est le ratio massique du débit total de solvant injecté sur le débit total d'éthylène gazeux injecté dans le procédé.

De préférence, les alpha oléfines linéaires obtenues comprennent de 4 à 20 atomes de carbone, de préférence de 4 à 18 atomes de carbone, de préférence de 4 à 10 atomes de carbone, et de préférence de 4 à 8 atomes de carbone. De manière préférée, les oléfines sont des alpha-oléfines linéaires, choisi parmi le but-1-ène, le hex-1-ène ou l'oct-1-ène.

Avantageusement, le procédé d'oligomérisation est mis en œuvre à une pression comprise entre 0,1 et 10,0 MPa, de préférence entre 0,2 et 9,0 MPa et préférentiellement entre 0,3 et 8,0 MPa, à une température comprise entre 30 et 200°C, de préférence entre 35 et 150°C et de manière préférée entre 45 et 140°C.

De préférence, la concentration en catalyseur dans le système catalytique est comprise entre 0,001 et 300,0 ppm en masse de métal atomique par rapport à la masse réactionnelle, de préférence entre 0,002 et 100,0 ppm, préférentiellement entre 0,003 et 50,0 ppm, plus préférentiellement entre 0,05 et 20,0 ppm et encore plus préférentiellement entre 0,1 et 10,0 ppm en masse de métal atomique par rapport à la masse réactionnelle.

Selon un mode de réalisation, le procédé d'oligomérisation est mis en œuvre en discontinu. On introduit le système catalytique, constitué comme décrit ci-dessus, dans un réacteur selon l'invention, avantageusement muni de chauffage et de refroidissement, puis on pressurise par de l'éthylène à la pression désirée, et on ajuste la température à la valeur souhaitée. La pression est maintenue constante dans le réacteur par introduction de la charge oléfinique gazeuse jusqu'à ce que le volume total de liquide produit représente, par exemple, de 1 à 1000 fois le volume de la solution catalytique préalablement introduite. On détruit alors le catalyseur par tout moyen habituel connu de l'homme du métier, puis on soutire et on sépare les produits de la réaction et le solvant.

Selon un autre mode de réalisation, le procédé d'oligomérisation est mise en œuvre en continu. Le système catalytique, constitué comme décrit ci-dessus, est injecté en même temps que la charge oléfinique gazeuse, de préférence l'éthylène dans un réacteur selon l'invention, et maintenu à la température souhaitée. On peut aussi injecter séparément les composants du système catalytique dans le milieu réactionnel. La charge oléfinique gazeuse, de préférence l'éthylène gazeux, est introduit par une vanne d'admission asservie à la pression, qui maintient celle-ci constante dans le réacteur. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits issus de la réaction ainsi que le solvant sont séparés, par exemple par distillation. L'éthylène qui n'a pas été transformé peut être recyclé dans le réacteur. Les résidus de catalyseur inclus dans une fraction lourde peuvent être incinérés.

### EXEMPLES

Les exemples ci-dessous illustrent l'invention sans en limiter la portée.

### Exemple 1 (comparatif) :

Le procédé d'oligomérisation de l'éthylène est mis en oeuvre dans un réacteur de type colonne à bulles. Le réacteur est opéré à une pression de 5,0 MPa et à une température de 120°C. Le volume réactionnel se compose, conformément à la figure 1, en deux zones A et B, en une colonne de 2,97 m de diamètre et de 6,0 m de hauteur liquide, et d'une boucle de recirculation ayant un volume total de 5,0 m³.

La colonne est équipée d'un dispositif d'injection d'éthylène gazeux, situé à 1,0 m du fond de la colonne.

Le système catalytique introduit dans l'enceinte réactionnelle est un système catalytique à base de chrome à une teneur de 5,2 ppm en chrome, tel que décrit dans le brevet FR3019064, en présence de cyclohexane comme solvant.

Le débit de purge est égal à 0,0045 kg/s.

La productivité volumique de ce réacteur est de 0,13 tonnes d'héxène-1 produit par heure et par m³ de volume réactionnel.

Les performances de ce réacteur permettent d'avoir une saturation en éthylène dissout de 52,6 %.

La production en héxène-1 est de 6,25 tonnes/heure, la sélectivité en héxène-1 est de 80,5 % poids, et le temps de séjour dans le réacteur est de 78,5 minutes, pour un taux massique de solvant de 1,0. Ledit taux massique de solvant est calculé comme le ratio massique du débit de solvant injecté sur le débit d'éthylène gazeux injecté.

### Exemple 2 (selon l'invention) :

Le procédé d'oligomérisation de l'éthylène est mis en oeuvre dans un réacteur gaz/liquide de type colonne à bulles. Le réacteur est opéré à une pression de 5,0 MPa et à une température de 120°C. Le volume réactionnel se compose, conformément à la figure 2, en deux zones A et B, en une colonne de 2,97 m de diamètre et de 6,0 m de hauteur liquide, et d'une boucle de recirculation ayant un volume total de 5,0 m³.

La colonne est équipée, selon l'invention, d'un dispositif d'injection d'éthylène gazeux et d'un dispositif d'injection des produits liquides, ce qui permet de réduire la taille initiale des bulles d'éthylène d'un facteur 5. Ces distributeurs sont situés à 1,0 m du fond de la colonne.

Le système catalytique introduit dans l'enceinte réactionnelle est un système catalytique à base de chrome à une teneur de 3,0 ppm en chrome, tel que décrit dans le brevet FR3019064, en présence de cyclohexane comme solvant.

Le débit de purge est égal à 0,0045 kg/s.

La productivité volumique de ce réacteur est la même que dans l'exemple précédent.

Les performances de ce réacteur permettent d'avoir une saturation en éthylène dissout de 90,5 %.

La production en héxène-1 est de 6,25 tonnes/heure, la sélectivité en héxène-1 est de 83.3 % poids, et le temps de séjour dans le réacteur est de 69,8 minutes, pour un taux massique de solvant de 1,0. Ledit taux de solvant est calculé comme le ratio massique du débit de solvant injecté sur le débit d'éthylène gazeux injecté.

Le dispositif selon l'invention permet ainsi d'améliorer la saturation de l'éthylène de 37,9 %, et donc la sélectivité en alpha-oléfine de 2,8 % en réduisant la consommation catalytique (72 % sur la concentration catalytique) et donc le coût opératoire du procédé, par rapport au cas selon l'art antérieur.

## Revendications

1. Procédé d'oligomérisation d'une charge oléfinique gazeuse comprenant la mise en contact d'un liquide comprenant un système catalytique comprenant un catalyseur métallique, au moins un activateur et au moins un additif, et éventuellement un solvant, et de la charge oléfinique gazeuse au moyen d'un dispositif d'injection de gaz et d'un dispositif d'injection de liquide, lesdits dispositifs d'injection étant agencés de manière à ce que l'injection du liquide entraine une diminution par cisaillement de la taille des bulles d'éthylène gazeux, ledit procédé mettant en œuvre un réacteur gaz/liquide d'oligomérisation d'une charge oléfinique gazeuse comprenant un dispositif d'injection de gaz (3) comprenant au moins un orifice d'injection de gaz et un dispositif d'injection de liquide (12) comprenant au moins un orifice d'injection de liquide, chaque orifice d'injection de gaz étant situé à proximité d'un orifice du dispositif d'injection de liquide (11) et positionné de manière à ce que la trajectoire d'injection du gaz soit dans le plan de la trajectoire d'injection du liquide, lesdits dispositifs d'injection étant agencés de manière à ce que l'injection du liquide puisse entrainer une diminution par cisaillement de la taille des bulles lors de l'injection de la charge oléfinique gazeuse.

2. Procédé selon la revendication 1 dans lequel les orifices d'injection du liquide et les orifices d'injection de gaz sont de formes circulaires, et dans lequel les orifices d'injection du liquide présentent un diamètre supérieur ou égal au diamètre des orifices d'injection de gaz.

3. Procédé selon l'une quelconque des revendications précédentes dans lequel au moins un orifice d'injection de gaz et au moins un orifice d'injection de liquide sont positionnés en regard l'un par rapport à l'autre selon un angle compris entre 0° et 180°.

4. Procédé selon l'une quelconque des revendications précédentes dans lequel les dispositifs d'injection de gaz et d'injection de liquide sont choisis parmi une conduite, un réseau de conduites, un distributeur multitubulaire, une plaque perforée, un tube cylindrique, un tube concentrique.

5. Procédé selon la revendication précédente dans lequel le dispositif d'injection de gaz est un tube cylindrique ayant une forme d'anneau circulaire présentant des orifices d'injection et le dispositif d'injection de liquide est un tube cylindrique ayant une forme d'anneau circulaire présentant des orifices d'injection.

6. Procédé selon la revendication précédente dans lequel le dispositif d'injection de gaz sous forme d'anneau circulaire présente un diamètre inférieur à celui du dispositif d'injection de liquide sous forme d'anneau circulaire, et dans lequel ledit dispositif d'injection de gaz se positionne à l'intérieur du dispositif d'injection de liquide sur un plan différent.

7. Procédé selon la revendication précédente dans lequel un enchainement de plusieurs dispositifs d'injection de liquide et de gaz sous forme d'anneau circulaire de diamètres décroissants sont alternés de la périphérie vers le centre représenté par l'axe central du dispositif ayant le diamètre le plus grand, lesdits dispositifs étant positionnés de sorte qu'un orifice d'injection de gaz d'un dispositif d'injection de gaz soit positionné à proximité d'un orifice du dispositif d'injection de liquide adjacent de manière à ce que la trajectoire d'injection de liquide soit dans le même plan de la trajectoire d'injection du gaz afin de provoquer le cisaillement dudit gaz.

8. Procédé selon l'une quelconque des revendications précédentes dans lequel les orifices des dispositifs d'injection de gaz et de liquide sont prolongés chacun par un tube (13, 15).

9. Procédé selon la revendication précédente dans lequel le ou les tubes (13) du dispositif d'injection de gaz (3) présente un diamètre inférieur à celui du ou des tubes (15) d'injection de liquide (12) et l'extrémité ouverte de sortie du tube (13) du dispositif d'injection de gaz (3) est positionnée à l'intérieur du tube d'injection de liquide de manière coaxiale.

10. Procédé selon l'une quelconque des revendications précédentes dans lequel le tube d'injection de liquide (15) de liquide comprend un déflecteur.

11. Procédé selon l'une quelconque des revendications précédentes dans lequel l'extrémité du tube d'injection de liquide (15) présente un rétrécissement du diamètre de sortie.

12. Procédé selon l'une quelconque des revendications précédentes comprenant
- une enceinte réactionnelle, de forme allongée le long d'un axe vertical, pouvant contenir
une phase liquide située dans une zone inférieure comprenant, et de préférence constituée, des produits de la réaction, de la charge oléfinique gazeuse et dissoute, de préférence de l'éthylène dissout et gazeux, d'un système catalytique et d'un éventuel solvant, et une phase gazeuse située dans une zone supérieure au-dessus de la zone inférieure comprenant de la charge oléfinique gazeuse et de préférence de l'éthylène gazeux, ainsi que des gaz incondensables (éthane notamment),
- un moyen d'introduction du système catalytique, ledit moyen étant situé dans la partie inférieure de l'enceinte réactionnelle,
- une boucle de recirculation comprenant un moyen de soutirage à la base (de préférence au fond) de l'enceinte réactionnelle pour le soutirage d'une fraction liquide, un échangeur thermique permettant le refroidissement dudit liquide, et un moyen d'introduction dudit liquide refroidi, ledit moyen d'introduction étant situé dans la partie supérieure de la zone inférieure de l'enceinte réactionnelle,
- et éventuellement une boucle de recycle de la phase gazeuse pour recycler au moins une fraction de la phase gazeuse vers la zone inférieure de la phase liquide, comprenant un moyen de soutirage situé dans la zone supérieure de l'enceinte réactionnelle pour permettre le soutirage d'une fraction gazeuse au niveau de la phase gazeuse et un moyen d'introduction positionné dans la zone inférieure de l'enceinte réactionnelle pour permettre d'introduire ladite fraction gazeuse soutirée dans la phase liquide.

13. Procédé d'oligomérisation selon l'une quelconque des revendications précédentes dans lequel la charge oléfinique gazeuse comprend entre 2 et 6 atomes de carbone, de préférence entre 2 et 4 atomes de carbone.

14. Procédé d'oligomérisation selon l'une quelconque des revendications précédentes dans lequel la vitesse d'injection du liquide est supérieure à la vitesse d'injection de la charge oléfinique gazeuse de manière à favoriser le cisaillement de la taille des bulles d'oléfines gazeuses en bulles gazeuses de taille plus petite.

## Patentansprüche

1. Verfahren zur Oligomerisierung eines gasförmigen olefinischen Einsatzmaterials, umfassend das Inkontaktbringen einer Flüssigkeit, die ein katalytisches System umfasst, das einen metallischen Katalysator, mindestens einen Aktivator und mindestens ein Additiv und gegebenenfalls ein Lösungsmittel umfasst, und des gasförmigen olefinischen Einsatzmaterials mittels einer Gaseinspritzvorrichtung und einer Flüssigkeitseinspritzvorrichtung, wobei die Einspritzvorrichtungen so gestaltet sind, dass das Einspritzen der Flüssigkeit zu einer Verringerung durch Scherung der Größe der gasförmigen Ethylenblasen führt, wobei das Verfahren einen Gas/Flüssigkeits-Reaktor zur Oligomerisierung eines gasförmigen olefinischen Einsatzmaterial einsetzt, der eine Gaseinspritzvorrichtung (3) umfasst, die mindestens eine Gaseinspritzöffnung umfasst, und eine Flüssigkeitseinspritzvorrichtung (12), die mindestens eine Flüssigkeitseinspritzöffnung umfasst, wobei jede Gaseinspritzöffnung in der Nähe einer Öffnung der Flüssigkeitseinspritzvorrichtung (11) gelegen ist und so angeordnet ist, dass der Einspritzweg des Gases in der Ebene des Einspritzwegs der Flüssigkeit liegt, wobei die Einspritzvorrichtungen so gestaltet sind, dass das Einspritzen der Flüssigkeit zu einer Verringerung durch Scherung der Größe der Blasen beim Einspritzen des gasförmigen olefinischen Einsatzmaterials führen kann.

2. Verfahren nach Anspruch 1, wobei die Einspritzöffnungen der Flüssigkeit und die Gaseinspritzöffnungen kreisförmig sind und wobei die Einspritzöffnungen der Flüssigkeit einen Durchmesser aufweisen, der größer als oder gleich dem Durchmesser der Gaseinspritzöffnungen ist.

3. Verfahren nach einem der vorhergehenden Ansprüche, wobei mindestens eine Gaseinspritzöffnung und mindestens eine Flüssigkeitseinspritzöffnung einander gegenüber in einem Winkel zwischen 0° und 180° angeordnet sind.

4. Verfahren nach einem der vorhergehenden Ansprüche, wobei die Gaseinspritz- und Flüssigkeitseinspritzvorrichtungen unter einer Leitung, einem Leitungsnetz, einem Mehrrohrverteiler, einer Lochplatte, einem zylindrischen Rohr, einem konzentrischen Rohr ausgewählt sind.

5. Verfahren nach dem vorhergehenden Anspruch, wobei die Gaseinspritzvorrichtung ein zylindrisches Rohr mit einer Kreisringform ist, das Einspritzöffnungen aufweist, und die Flüssigkeitseinspritzvorrichtung ein zylindrisches Rohr mit einer Kreisringform ist, das Einspritzöffnungen aufweist.

6. Verfahren nach dem vorhergehenden Anspruch, wobei die kreisringförmige Gaseinspritzvorrichtung einen Durchmesser aufweist, der kleiner als derjenige der kreisringförmigen Flüssigkeitseinspritzvorrichtung ist, und wobei die Gaseinspritzvorrichtung im Inneren der Flüssigkeitseinspritzvorrichtung auf einer unterschiedlichen Ebene angeordnet ist.

7. Verfahren nach dem vorhergehenden Anspruch, wobei eine Verkettung von mehreren kreisringförmigen Flüssigkeits- und Gaseinspritzvorrichtungen mit abnehmenden Durchmessern vom Umfang zum Mittelpunkt hin, der durch die zentrale Achse der Vorrichtung mit dem größten Durchmesser repräsentiert wird, alternieren, wobei die Vorrichtungen so angeordnet sind, dass eine Gaseinspritzöffnung einer Gaseinspritzvorrichtung in der Nähe einer Öffnung der benachbarten Flüssigkeitseinspritzvorrichtung angeordnet ist, so dass der Flüssigkeitseinspritzweg in derselben Ebene des Einspritzwegs des Gases liegt, um die Scherung des Gases zu bewirken.

8. Verfahren nach einem der vorhergehenden Ansprüche, wobei die Öffnungen der Gas- und Flüssigkeitseinspritzöffnungen jeweils durch ein Rohr (13, 15) verlängert werden.

9. Verfahren nach dem vorhergehenden Anspruch, wobei das oder die Rohre (13) der Gaseinspritzvorrichtung (3) einen Durchmesser aufweist, der kleiner ist als derjenige des oder der Rohre (15) zum Einspritzen von Flüssigkeit (12), und das offene Austrittsende des Rohrs (13) der Gaseinspritzvorrichtung (3) koaxial im Inneren des Flüssigkeitseinspritzrohrs angeordnet ist.

10. Verfahren nach einem der vorhergehenden Ansprüche, wobei das Flüssigkeitseinspritzrohr (15) für Flüssigkeit einen Deflektor umfasst.

11. Verfahren nach einem der vorhergehenden Ansprüche, wobei das Ende des Flüssigkeitseinspritzrohrs (15) eine Verengung des Austrittsdurchmessers aufweist.

12. Verfahren nach einem der vorhergehenden Ansprüche, umfassend
- eine Reaktionskammer von länglicher Form entlang einer vertikalen Achse, die enthalten kann
eine in einem unteren Bereich gelegene flüssige Phase, die Produkte der Reaktion, des gasförmigen und gelösten olefinischen Einsatzmaterials, bevorzugt des gelösten und gasförmigen Ethylens, eines katalytischen Systems und gegebenenfalls eines Lösungsmittels umfasst und bevorzugt daraus besteht, und eine in einem oberen Bereich über dem unteren Bereich gelegene gasförmige Phase, die gasförmiges olefinisches Einsatzmaterial und bevorzugt gasförmiges Ethylen sowie unkondensierbare Gase (insbesondere Ethan) umfasst,
- ein Mittel zum Einleiten des katalytischen Systems, wobei das Mittel in dem unteren Teil der Reaktionskammer gelegen ist,
- einen Rezirkulationskreis, umfassend ein Abzugsmittel an der Unterseite (bevorzugt am Boden) der Reaktionskammer zum Abziehen einer flüssigen Fraktion, einen Wärmetauscher, der das Kühlen der Flüssigkeit ermöglicht, und ein Mittel zum Einleiten der gekühlten Flüssigkeit, wobei das Mittel zum Einleiten in dem oberen Teil des unteren Bereichs der Reaktionskammer gelegen ist,
- und gegebenenfalls einen Rezyklatkreis der gasförmigen Phase, um mindestens eine Fraktion der gasförmigen Phase zu dem unteren Bereich der flüssigen Phase hin zurückzuführen, umfassend ein Abzugsmittel, das in dem oberen Bereich der Reaktionskammer gelegen ist, um das Abziehen einer gasförmigen Fraktion an der gasförmigen Phase zu ermöglichen, und ein Einleitungsmittel, das in dem unteren Bereich der Reaktionskammer angeordnet ist, um das Einleiten der abgezogenen gasförmigen Fraktion in die flüssige Phase zu ermöglichen.

13. Verfahren zur Oligomerisierung nach einem der vorhergehenden Ansprüche, wobei das gasförmige olefinische Einsatzmaterial zwischen 2 und 6 Kohlenstoffatome, vorzugsweise zwischen 2 und 4 Kohlenstoffatome umfasst.

14. Verfahren zur Oligomerisierung nach einem der vorhergehenden Ansprüche, wobei die Einspritzgeschwindigkeit der Flüssigkeit höher als die Einspritzgeschwindigkeit des gasförmigen olefinischen Einsatzmaterials ist, so dass die Scherung der Größe der gasförmigen Olefinblasen zu Gasblasen mit geringerer Größe begünstigt wird.

## Claims

1. Process for the oligomerization of a gaseous olefinic feedstock comprising bringing a liquid comprising a catalytic system comprising a metal catalyst, at least one activator and at least one additive, and optionally a solvent, into contact with the gaseous olefinic feedstock by means of a gas injection device and of a liquid injection device, said injection devices being arranged so that the injection of the liquid brings about a reduction by shearing in the size of the gaseous ethylene bubbles, said process employing a gas/liquid reactor for the oligomerization of a gaseous olefinic feedstock comprising a gas injection device (3) comprising at least one gas injection orifice and a liquid injection device (12) comprising at least one liquid injection orifice, each gas injection orifice being located close to an orifice of the liquid injection device (11) and positioned so that the injection trajectory of the gas is in the plane of the injection trajectory of the liquid, said injection devices being arranged so that the injection of the liquid can bring about a reduction by shearing in the size of the bubbles during the injection of the gaseous olefinic feedstock.

2. Process according to Claim 1, in which the liquid injection orifices and the gas injection orifices are circular in shape, and in which the liquid injection orifices exhibit a diameter which is greater than or equal to the diameter of the gas injection orifices.

3. Process according to either one of the preceding claims, in which at least one gas injection orifice and at least one liquid injection orifice are positioned facing each other along an angle of between 0° and 180°.

4. Process according to any one of the preceding claims, in which the gas injection and liquid injection devices are chosen from a pipe, a network of pipes, a multitubular distributor, a perforated plate, a cylindrical tube and a concentric tube.

5. Process according to the preceding claim, in which the gas injection device is a cylindrical tube having a circular ring form exhibiting injection orifices and the liquid injection device is a cylindrical tube having a circular ring form exhibiting injection orifices.

6. Process according to the preceding claim, in which the gas injection device in circular ring form exhibits a diameter which is less than that of the liquid injection device in circular ring form, and in which said gas injection device is positioned inside the liquid injection device on a different plane.

7. Process according to the preceding claim, in which a sequence of several liquid and gas injection devices in circular ring form of decreasing diameters are alternated from the periphery towards the centre represented by the central axis of the device having the greatest diameter, said devices being positioned so that a gas injection orifice of a gas injection device is positioned close to an orifice of the adjacent liquid injection device so that the injection trajectory of the liquid is in the same plane of the injection trajectory of the gas in order to bring about the shearing of said gas.

8. Process according to any one of the preceding claims, in which the orifices of the gas and liquid injection devices are each extended by a tube (13, 15).

9. Process according to the preceding claim, in which the tube or tubes (13) of the gas injection device (3) exhibit a diameter which is smaller than that of the tube or tubes (15) for injection of liquid (12) and the open outlet end of the tube (13) of the gas injection device (3) is positioned coaxially inside the tube for injection of liquid.

10. Process according to any one of the preceding claims, in which the liquid injection tube (15) comprises a deflector.

11. Process according to any one of the preceding claims, in which the end of the liquid injection tube (15) exhibits a narrowing of the outlet diameter.

12. Process according to any one of the preceding claims, comprising
- a reaction chamber, of elongated shape along a vertical axis, which can contain
a liquid phase located in a lower zone, comprising and preferably consisting of the products of the reaction, of the dissolved and gaseous olefinic feedstock, preferably of dissolved and gaseous ethylene, of a catalytic system and of an optional solvent, and a gas phase located in an upper zone above the lower zone, comprising gaseous olefinic feedstock and preferably gaseous ethylene, and also non-condensable gases (in particular ethane),
- a means for introduction of the catalytic system, said means being located in the lower part of the reaction chamber,
- a recirculation loop comprising a withdrawal means at the base (preferably at the bottom) of the reaction chamber for the withdrawal of a liquid fraction, a heat exchanger making the cooling of said liquid possible, and a means for introduction of said cooled liquid, said introduction means being located in the upper part of the lower zone of the reaction chamber,
- and optionally a recycle loop for the gas phase for recycling at least a fraction of the gas phase to the lower zone of the liquid phase, comprising a withdrawal means located in the upper zone of the reaction chamber to make the withdrawal of a gas fraction at the level of the gas phase possible and an introduction means positioned in the lower zone of the reaction chamber to make it possible to introduce said withdrawn gas fraction into the liquid phase.

13. Oligomerization process according to any one of the preceding claims, in which the gaseous olefinic feedstock comprises between 2 and 6 carbon atoms, preferably between 2 and 4 carbon atoms.

14. Oligomerization process according to any one of the preceding claims, in which the rate of injection of the liquid is greater than the rate of injection of the gaseous olefinic feedstock so as to promote the shearing in the size of the bubbles of gaseous olefins to give gas bubbles of smaller size.
